# EUROPEAN PATENT APPLICATION

(11) **EP 4 834 318 A1**
(43) Date of publication of application: **04.11.2026**
(21) Application number: 24912392.8
(22) Date of filing: 12.12.2024
(51) Int. Cl.: A61K 8/49, A61Q 19/00

(54) **PUFFINESS-REDUCING AGENT AND COSMETIC**

(30) Priority: 26.12.2023 JP 2023220018
(71) Applicant: Shiseido Company, Ltd., Chuo-ku Tokyo 104-0061 (JP)
(72) Inventor: TAKAI, Eisuke, Tokyo 104-0061 (JP)
(74) Representative: Ter Meer und Partner mbB
(86) International application number: PCT/JP2024/043918
(87) International publication number: WO 2025/142503

(57) **Abstract**

The puffiness-reducing agent according to one embodiment of the present disclosure includes an active ingredient that is 1-(2-hydroxyethyl)-2-imidazolidinone or a derivative of 1-(2-hydroxyethyl)-2-imidazolidinone.

## Description

### TECHNICAL FIELD

The present invention relates to puffiness-reducing agents and cosmetics.

### BACKGROUND ART

The cutaneous vasculature is located in the dermis, and is made up of blood vessels and lymph vessels. In order to maintain homeostasis, the tissue fluid that has seeped from a blood vessel needs to be returned back into veins. The veins in the skin efficiently lead the blood flow to the central nervous system. However, the veins themselves have a poor capability of taking up the tissue fluid. Therefore, it has been understood that tissues that take in the tissue fluid, i.e., lymph vessels, are also an essential structure in the skin.

The lymph vessels play an important role in maintaining the microenvironment around cells in a stable state by collecting unwanted substances present in the skin, or collecting water and proteins constantly leaking from blood vessels. A state, in which water or the like leaked from blood vessels is not smoothly drained into veins or lymph, and more than a necessary amount of the water or the like is retained between cells, is generally described as "puffiness".

For example, puffiness of the body, such as a puffy face when waking up, puffy feet when tired, or the like, is not only undesirable for health, but is also adversarial to beauty. In addition, if puffiness is left as it is, the blood vessels in the surrounding area are compressed, and the symptom of puffiness further progresses, leading to pathological swelling.

As a method for eliminating puffiness, there has been a method in which circulation of the blood and lymph is improved by massage or the like to smoothly discharge water. In addition, it has been known that drugs, plant extracts, or the like are effective for reducing puffiness. For example, technologies, such as a puffiness-reducing agent including, as an active ingredient, α-glucosylrutin (see Patent Document 1), a puffiness-reducing agent including, as an active ingredient, α-glucosylhesperidin (see Patent Document 2), a puffiness-reducing agent including, as an active ingredient, Poria Sclerotium (see Patent Document 3), a skin puffiness-reducing agent including at least one selected from the group consisting of extracts of plants belonging to Genus Bupleurum, extracts of plants belonging to Genus Coix, extracts of plants belonging to Genus Zea, and whey extracts (see Patent Document 4), a puffiness-reducing agent including at least one selected from the group consisting of extracts of plants belonging to Genus Acorus (e.g., Acoruscalamus L., and Acorus gramineus Soland), Genus Hamamelis (e.g., Hamamelisvirginiana L.), Genus Citrus (e.g., Citrus aurantium L.), Genus Zanthoxylum (e.g., Zanthoxylumpiperitum L. DC.), Genus Carica (e.g., Carica papaya L.), Genus Vitis (e.g., Vitisvinifera L.), Genus Thymus (e.g., Thymus serpyllum L.), Genus Pinus (e.g., Pinussylvestris L.), and Genus Salvia (e.g., Salvia splendens Sellow) (see Patent Document 5), and the like are disclosed.

Although numerous attempts have been made as described above, a sufficiently satisfactory puffiness reduction effect has not been achieved, and therefore there is a strong demand for a puffiness-reducing agent that can reduce puffiness more effectively.

### CITATION LIST

### PATENT DOCUMENT

Patent Document 1: Japanese Unexamined Patent Application Publication No. H10-182468
Patent Document 2: Japanese Unexamined Patent Application Publication No. H10-218777
Patent Document 3: Japanese Unexamined Patent Application Publication No. 2000-239138
Patent Document 4: Japanese Unexamined Patent Application Publication No. 2003-14739
Patent Document 5: Japanese Unexamined Patent Application Publication No. 2004-35425

### SUMMARY OF THE INVENTION

### TECHNICAL PROBLEM

Considering the above, one aspect of the present invention aims to provide a puffiness-reducing agent having an excellent puffiness reduction effect.

### SOLUTION TO THE PROBLEM

In order to solve the above problems, one aspect of the puffiness-reducing agent according to the present invention includes an active ingredient that is 1-(2-hydroxyethyl)-2-imidazolidinone or a derivative of 1-(2-hydroxyethyl)-2-imidazolidinone.

### EFFECTS OF THE INVENTION

According to one aspect of the present invention, there can be provided a puffiness-reducing agent having an excellent puffiness reduction effect.

### BRIEF DESCRIPTION OF THE DRAWINGS

[Fig. 1] Fig. 1 is a diagram for explaining gravitational swelling in order to evaluate a puffiness reduction effect of the puffiness-reducing agent according to one embodiment.
[Fig. 2] Fig. 2 is a diagram for explaining gravitational shrinking in order to evaluate the puffiness reduction effect of the puffiness-reducing agent according to one embodiment.
[Fig. 3A] Fig. 3A is a schematic explanatory diagram of a pair of left and right face photographs in Test Example 1.
[Fig. 3B] Fig. 3B is a graph depicting the results of the visual sensory determination in Test Example 1. The vertical axis represents the result of the visual sensory determination, and the horizontal axis represents the execution time of the visual sensory determination.
[Fig. 4] Fig. 4 is a graph depicting a change in reduction of an amount of puffiness (Sc) of the face at Week 0 to 6 weeks later from the start of the test in Test Example 1. The vertical axis represents the reduction (cc) of the amount of the puffiness (Sc), and the horizontal axis represents the test period (week).

### DETAILED DESCRIPTION OF THE EMBODIMENTS

Hereinafter, embodiments of the present invention will be described in detail. The embodiments are not limited to the following description, and can be appropriately modified without departing from the gist of the present invention. In addition, in the present specification, a numerical range indicated by "to" means that numerical values described before and after "to" are included as a lower limit and an upper limit, unless otherwise specified.

### (Puffiness-reducing agent)

The puffiness-reducing agent according to one embodiment includes an active ingredient that is 1-(2-hydroxyethyl)-2-imidazolidinone or a derivative of 1-(2-hydroxyethyl)-2-imidazolidinone, and may further include other ingredients, as necessary.

The "puffiness" refers to a state in which water in blood leaks out from blood vessels for some reason, and water is abnormally accumulated in the skin or subcutaneous tissues. The term "puffiness" may be also referred to as "edema".

A method of confirming the puffiness reduction effect of the puffiness-reducing agent according to one embodiment is not particularly limited, and may be appropriately selected according to the intended purpose. For example, when reduction in puffiness can be observed after application of the puffiness-reducing agent by a sensory evaluation, as compared with a state before the application of the puffiness-reducing agent or a state with no puffiness-reducing agent being applied, it can be determined that the puffiness-reducing agent has a puffiness reduction effect.

In addition, a method of confirming the presence or absence of puffiness by pressing with a finger can also be used. Such a method has been performed for the limbs, particularly the feet. Specifically, before the application of the puffiness-reducing agent or with no puffiness-reducing agent being applied, a target region of the skin is pressed with the finger pad for 5 seconds, and the time (referred to as "T0" hereinafter) required for the skin to return back to the original state from the indentation state after releasing the finger is measured. Next, after the application of the puffiness-reducing agent, the same target region of the skin to which the puffiness-reducing agent has been applied is pressed with the finger pad for 5 seconds, and the time (referred to as "T1" hereinafter) required for the skin to return back to the original state from the indentation state after releasing the finger is measured. In the case where T1 is shorter than T0, i.e., (T1-T0) is a positive value, it can be determined that the puffiness-reducing agent has a puffiness reduction effect.

Further, in the case where puffiness of the face is determined, a puffiness reduction effect can be confirmed by the following quantitative method. Specifically, before application of the puffiness-reducing agent or with no puffiness-reducing agent being applied to a subject, data representing a three-dimensional shape of the face of the subject (may be referred to as a "three-dimensional face shape image" hereinafter) is captured using a known three-dimensional imaging device. At the time of capturing data, three-dimensional face shape images are obtained with the face of the subject being in the horizontal position (specifically, the median line of the face is fixed to be orthogonal to the direction of gravity), and with the face of the subject being in the vertical position (specifically, the median line of the face is fixed to be parallel to the direction of gravity), respectively. Next, an amount of gravitational swelling (VCswelling) and an amount of gravitational shrinking described below are calculated using a computer, based on the change in the three-dimensional shape between the three-dimensional face shape image with the face of the subject being in the horizontal position and the three-dimensional face shape image with the face of the subject being in the vertical position. Next, an amount of puffiness (Sc) of the face described below is calculated based on the amount of gravitational swelling (VCswelling) and the amount of gravitational shrinking (VCshrinking). Similarly, after applying the puffiness-reducing agent to the subject, an amount of gravitational swelling amount (VCswelling), an amount of gravitational shrinking amount (VCshrinking), and an amount of puffiness (Sc) of the face are calculated from the data representing the three-dimensional shape of the face of the subject. Next, the amount of the puffiness (Sc) of the face before the application of the puffiness-reducing agent or in with no puffiness-reducing agent being applied is compared with the amount of the puffiness (Sc) of the face after the application of the puffiness-reducing agent, and it can be determined whether the puffiness-reducing agent has a puffiness reduction effect.

### -Calculation of amount of gravitational swelling (VCswelling)-

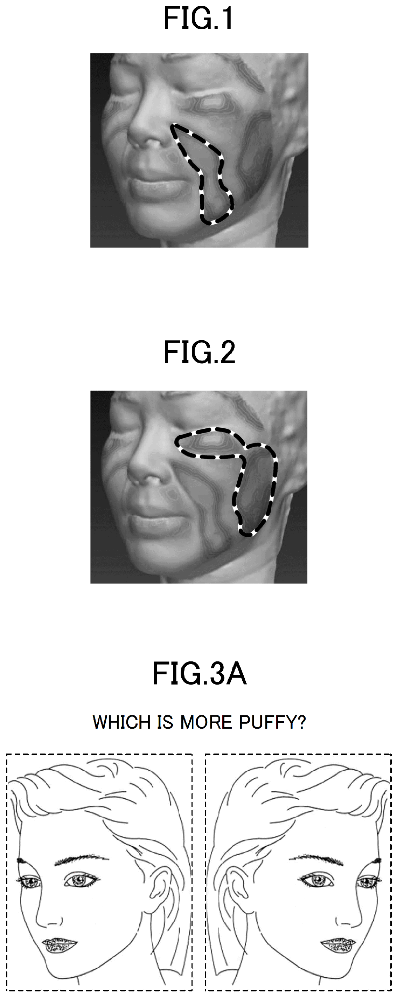

Fig. 1 is a diagram for explaining the gravitational swelling according to one embodiment of the present invention. A three-dimensional face shape image with the face of the subject being in the horizontal position, and a three-dimensional face shape image with the face of the subject being in the vertical position are acquired, and the three-dimensional shape in the horizontal position and the three-dimensional shape in the vertical position are compared. As a result, the volume of the site indicated by the dashed line in Fig. 1 is larger in the vertical position. In this manner, the three-dimensional shape of the subject in the horizontal position and the three-dimensional shape of the subject in the vertical position are compared, and a difference in the volume of the site, which has a larger volume in the vertical position, between both positions (i.e., the horizontal position and the vertical position) is determined as an amount of gravitational swelling (VCswelling).

### -Calculation of amount of gravitational shrinking (VCshrinking)-

Fig. 2 is a diagram for explaining gravitational shrinking according to one embodiment of the present invention. A three-dimensional face shape image with the face of the subject being in the horizontal position and a three-dimensional face shape image with the face of the subject being in the vertical position are acquired, and the three-dimensional shape in the horizontal position and the three-dimensional shape in the vertical position are compared. As a result, the volume of the site indicated by the dashed line in Fig. 2 is smaller in the vertical position. In this manner, a difference in the volume of the site, which has a smaller volume in the vertical position, between both positions (i.e., the horizontal position and the vertical position) is determined as an amount of gravitational shrinking (VCshrinking).

### -Calculation of amount of puffiness (Sc) of face-

The amount of puffiness (Sc) of the face can be calculated according to the following equation (1).
[Math. 1] ${S}_{c} = \frac{1}{\sqrt{2}} \left({VC}_{swelling}-{VC}_{shirinking}\right)$ Equation (1) (In the equation (1), "Sc" is the amount of puffiness of the face, "VCswelling" is the amount of gravitational swelling, and "VCshrinking" is the amount of gravitational shrinking.)

When "Sc0" is the amount of puffiness of the face before the application of the puffiness-reducing agent or with no puffiness-reducing agent being applied and "Sc1" is the amount of puffiness of the face after the application of the puffiness-reducing agent, the reduction in the amount of puffiness (Sc) of the face can be calculated according to the following equation (2). In the case where the reduction in the amount of puffiness (Sc) of the face is a positive value, it can be confirmed that the puffiness-reducing agent has a puffiness reduction effect. In addition, the larger numerical value of the reduction in the amount of puffiness (Sc) indicates the better puffiness reduction effect of the puffiness-reducing agent. Further, the puffiness reduction effect of the puffiness-reducing agent can also be confirmed by the method described in Test Examples below.$Reduction in amount of puffiness \left(Sc\right) of face = Sc 0 - Sc 1$ Equation (2)

### <1-(2-hydroxyethyl)-2-imidazolidinone or derivative thereof>

1-(2-Hydroxyethyl)-2-imidazolidinone (may be referred to as "HEI" hereinafter) or a derivative of 1-(2-hydroxyethyl)-2-imidazolidinone is not particularly limited, and may be appropriately selected according to the intended purpose, as long as HEI or the derivative of HEI has a puffiness reduction effect. HEI or the derivative of HEI is preferably a cyclic carboxamide derivative represented by the following general formula (1) or a salt of the cyclic carboxamide derivative. 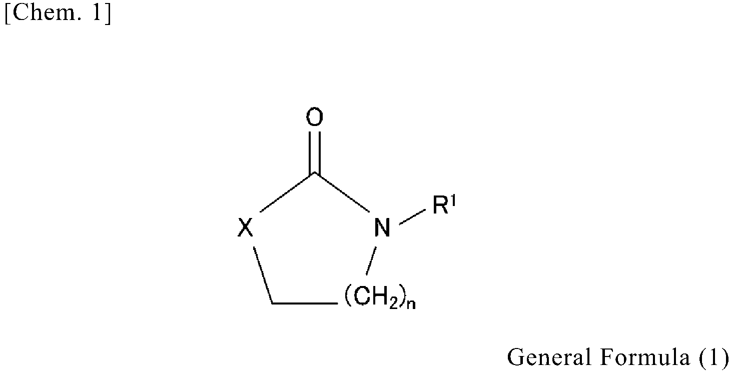 (In the general formula (1), n is an integer of 1 to 3, R¹ is a hydrogen atom or a hydrocarbon group that includes 1 to 6 carbon atoms and may be substituted with a hydroxyl group; X is a group represented by -CH₂- or -N(R²)-, where R² is a hydrogen atom or a hydrocarbon group that includes 1 to 6 carbon atoms and may be substituted with a hydroxyl group.)

In the general formula (1), n is an integer of 1 to 3, preferably an integer of 1 or 2, and more preferably an integer of 1.

In the case where R¹ is a hydrocarbon group that includes 1 to 6 carbon atoms and may be substituted with a hydroxy group in the general formula (1), the number of carbon atoms in the hydrocarbon group is preferably 1 to 4, more preferably 1 to 3, and yet more preferably 2.

In the case where X is a group represented by -N(R²)- in the general formula (1), R² is a hydrogen atom or a hydrocarbon group that includes 1 to 6 carbon atoms and may be substituted with a hydroxyl group, and is preferably a hydrogen atom.

Among the cyclic carboxamide derivatives represented by the general formula (1), a compound represented by the following structural formula (1) is 1-(2-hydroxyethyl)-2-imidazolidinone.

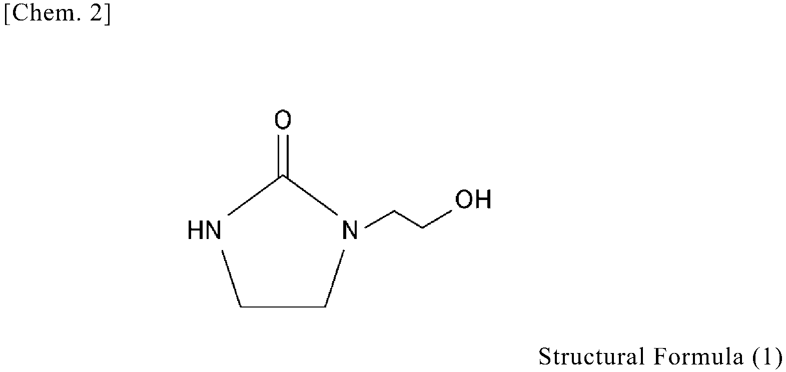

The derivative of 1-(2-hydroxyethyl)-2-imidazolidinone is any of the cyclic carboxamide derivatives represented by the general formula (1) other than the compound represented by the structural formula (1). The derivative of 1-(2-hydroxyethyl)-2-imidazolidinone is not particularly limited, and may be appropriately selected according to the intended purpose, as long as the derivative has a puffiness reduction effect. The derivative of 1-(2-hydroxyethyl)-2-imidazolidinone is preferably 2-imidazolidinone, which is a compound represented by the following structural formula (2), or 1-(2-hydroxyethyl)-2-pyrrolidone, which is a compound represented by the following structural formula (3).

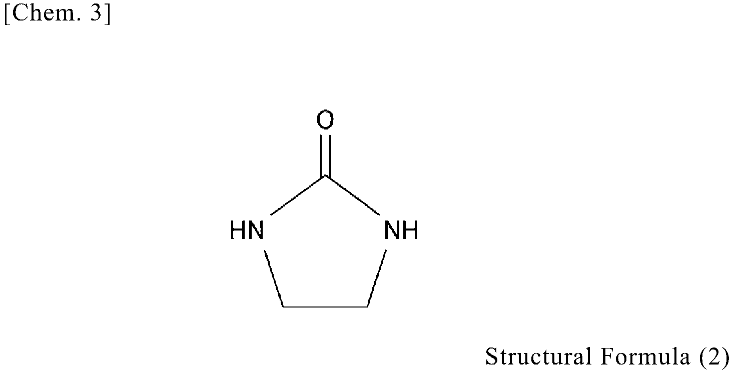

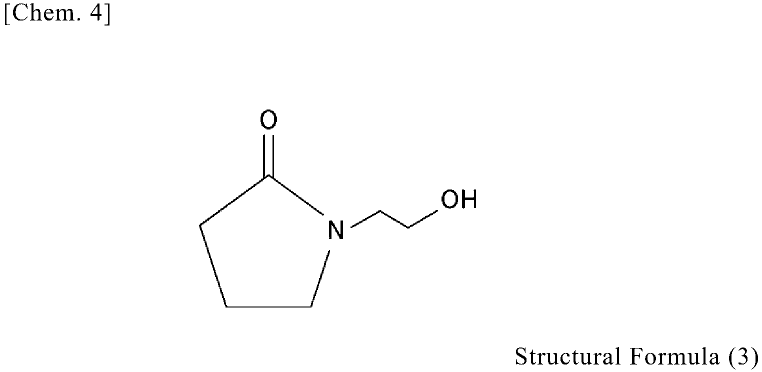

The salt of the cyclic carboxamide derivative represented by the general formula (1) is not particularly limited, and may be an inorganic salt or an organic salt.

The inorganic salt is not particularly limited, and may be appropriately selected according to the intended purpose. Examples of the inorganic salt include hydrochlorides, sulfates, phosphates, hydrobromides, sodium salts, potassium salts, magnesium salts, calcium salts, ammonium salts, and the like.

The organic salt is not particularly limited, and may be appropriately selected according to the intended purpose. Examples of the organic salt include acetates, lactates, maleates, fumarates, tartrates, citrates, methanesulfonates, p-toluenesulfonates, triethnol amine salts, diethanol amine salts, amino acid salts, and the like.

In the puffiness-reducing agent according to the one embodiment, as the 1-(2-hydroxyethyl)-2-imidazolidinone or the derivative of 1-(2-hydroxyethyl)-2-imidazolidinone, a cyclic carboxamide derivative represented by the general formula (1) or a salt thereof may be used alone, or two or more cyclic carboxamide derivatives or salts may be used in combination.

The 1-(2-hydroxyethyl)-2-imidazolidinone or the derivative of 1-(2-hydroxyethyl)-2-imidazolidinone may be appropriately synthesized according to a method known in the related art, or a commercial product may be used.

The amount of the 1-(2-hydroxyethyl)-2-imidazolidinone or the derivative of 1-(2-hydroxyethyl)-2-imidazolidinone in the puffiness-reducing agent according to one embodiment is not particularly limited, and may be appropriately selected according to the intended purpose, as long as the amount is sufficient enough to effectively exhibit a puffiness reduction effect. The amount of 1-(2-hydroxyethyl)-2-imidazolidinone or the derivative of 1-(2-hydroxyethyl)-2-imidazolidinone is preferably 0.0001 percent by mass or greater, more preferably 0.001 percent by mass or greater, further more preferably 0.01 percent by mass or greater, yet further more preferably 0.1 percent by mass or greater, and particularly preferably 1 percent by mass or greater. The upper limit of the amount of 1-(2-hydroxyethyl)-2-imidazolidinone or the derivative of 1-(2-hydroxyethyl)-2-imidazolidinone in the puffiness-reducing agent according to one embodiment is not particularly limited, and may be appropriately selected according to the intended purpose, as long as the amount is sufficient enough to effectively exhibit a puffiness reduction effect. The upper limit is preferably 5 percent by mass or less, more preferably 4 percent by mass or less, further more preferably 3 percent by mass or less, yet further more preferably 2 percent by mass or less, and particularly preferably 1.5 percent by mass or less, relative to the total mass of the puffiness-reducing agent. The lower limit and the upper limit of the amount of 1-(2-hydroxyethyl)-2-imidazolidinone or the derivative of 1-(2-hydroxyethyl)-2-imidazolidinone in the puffiness-reducing agent according to one embodiment can be appropriately combined, and are preferably 0.0001 percent by mass or greater and 5 percent by mass or less, more preferably 0.001 percent by mass or greater and 4 percent by mass or less, further more preferably 0.01 percent by mass or greater and 3 percent by mass or less, yet further more preferably 0.1 percent by mass or greater and 2 percent by mass or less, and particularly preferably 1 percent by mass or greater and 1.5 percent by mass or less relative to the total mass of the puffiness-reducing agent. In the case where the puffiness-reducing agent according to one embodiment includes two or more of 1-(2-hydroxyethyl)-2-imidazolidinone or the derivatives of 1-(2-hydroxyethyl)-2-imidazolidinone, the amount of 1-(2-hydroxyethyl)-2-imidazolidinone or the derivatives of 1-(2-hydroxyethyl)-2-imidazolidinone means a total amount of two or more of 1-(2-hydroxyethyl)-2-imidazolidinone or the derivatives of 1-(2-hydroxyethyl)-2-imidazolidinone.

### <Other ingredients>

The puffiness-reducing agent according to one embodiment may include other ingredients in addition to 1-(2-hydroxyethyl)-2-imidazolidinone or the derivative of 1-(2-hydroxyethyl)-2-imidazolidinone, as long as the other ingredients do not adversely affect a puffiness reduction effect. Examples of the other ingredients include pharmacologically acceptable carriers or adjuvants, such as diluents, binders, disintegrants, thickeners, dispersants, reabsorption promoters, flavoring agents, buffers, surfactants, solubilizers, preservatives, emulsifiers, isotonizing agents, stabilizers, pH adjusters, and the like. The above substances may be used alone or in combination of two or more.

The amount of the other ingredients in the puffiness-reducing agent according to one embodiment is not particularly limited, and may be appropriately selected according to the intended purpose, as long as a puffiness reduction effect is not adversely affected. The puffiness-reducing agent according to one embodiment may be composed of 1-(2-hydroxyethyl)-2-imidazolidinone or the derivative of 1-(2-hydroxyethyl)-2-imidazolidinone.

### [Administration method and administration dose]

The usage and dose of the puffiness-reducing agent according to one embodiment are not particularly limited, and may be appropriately selected according to the intended purpose, as long as a puffiness reduction effect can be exhibited. Examples of the administration method include a topical administration method, an oral administration method, a parenteral administration method (e.g., an intravenous administration method, an intraperitoneal administration method, etc.), and the like. The administration method is preferably a topical administration method, and more preferably a method in which the puffiness-reducing agent is used and applied onto skin.

The puffiness-reducing agent according to one embodiment can reduce puffiness preferably by applying the puffiness-reducing agent to the skin. The usage method and dose of the puffiness-reducing agent for the application to the skin are not particularly limited, and may be appropriately selected according to the foam of the puffiness-reducing agent, the age, body weight, or condition of puffiness of a subject, or the like. As the usage method, the puffiness-reducing agent is preferably applied a few times a day, and more preferably one time to five times a day. At the time of application, the puffiness-reducing agent may be directly rubbed onto skin, or a base material, such as a piece of gauze, may be impregnated with the puffiness-reducing agent, and the base material may then be adhered to the skin. The dose is preferably 0.001 mL to 1 mL per 1 cm² of skin.

### [Dosage form]

The dosage form of the puffiness-reducing agent according to one embodiment is not particularly limited, and may be appropriately selected according to the intended purpose. In the case where the puffiness-reducing agent is used as a topical dermatological agent by a topical administration method, examples of the dosage form include dosage forms, such as a solution form, a solubilized form, an emulsified form, a powder dispersion form, a water-oil two layer form, a water-oil-powder three layer form, an ointment, a cream, an emulsified lotion, a lotion, a gel, an aerosol, and the like. In addition, the puffiness-reducing agent may be prepared as a patch in which a base material is impregnated with any of the above dosage forms of the puffiness-reducing agent. In the case where the puffiness-reducing agent is used by an oral administration method, examples of the dosage form include: solid preparations, such as tablets, coated tablets, sugar-coated tablets, granules, powders, and capsules (e.g., hard and soft gelatin capsules, etc.); liquid preparations (solutions and suspensions), such as oral liquids and syrups; and the like. In the case where the puffiness-reducing agent is used by a parenteral administration method, examples of the dosage method include injection preparations and the like.

### [Production method]

A production method for the puffiness-reducing agent according to one embodiment is not particularly limited, and the puffiness-reducing agent can be produced by a method appropriately selected from known methods according to the form of the puffiness-reducing agent to be produced.

Use of 1-(2-hydroxyethyl)-2-imidazolidinone or the derivative of 1-(2-hydroxyethyl)-2-imidazolidinone for reducing puffiness and use of 1-(2-hydroxyethyl)-2-imidazolidinone or the derivative of 1-(2-hydroxyethyl)-2-imidazolidinone in production of the puffiness-reducing agent are also included within the scope of the present disclosure.

### (Cosmetic)

The cosmetic according to one embodiment includes the puffiness-reducing agent according to one embodiment, and may further include other ingredients, as necessary.

### <Puffiness-reducing agent>

The puffiness-reducing agent included in the cosmetic according to one embodiment is as described in the section of (Puffiness-reducing agent) above.

The amount of the puffiness-reducing agent in the cosmetic according to one embodiment is not particularly limited, and may be appropriately selected according to the intended purpose, as long as a puffiness reduction effect can be exhibited.

### <Other ingredients>

The cosmetic according to one embodiment may include other ingredients, in addition to the puffiness-reducing agent. The other ingredients are not particularly limited, and may be appropriately selected from ingredients typically formulated in cosmetics according to a form of the cosmetic or the like. Examples of the other ingredients include glycols, glycerins, sugar alcohols, saccharides, oily components, polymers, surfactants, powders, colorants, solvents, silicones, fragrances, drugs or medical ingredients other than the puffiness-reducing agent, pH adjusters, sequestering agents, and the like. The other ingredients may be used alone or in combination of two or more.

### <<Glycols>>

Examples of the glycols include propylene glycol, dipropylene glycol, 1,3-butylene glycol, 1,4-butylene glycol, diethylene glycol, triethylene glycol, polyethylene glycol, and the like.

### <<Glycerins>>

Examples of the glycerins include glycerin, diglycerin, polyglycerin, and the like.

### <<Sugar alcohols>>

Examples of the sugar alcohols include sorbitol, mannitol, maltitol, xylitol, erythritol, and the like.

### <<Saccharides>>

Examples of the saccharides include fructose, glucose, galactose, maltose, lactose, trehalose and the like.

### <<Oily components>>

Examples of the oily components include oil and fats, wax, hydrocarbon oil, higher fatty acids, higher alcohols, ester oil, silicone oil, oil-soluble drugs, and the like. The oily components may be used alone or in combination of two or more.

### -Oils and fats-

Examples of the oils and fats include: vegetable oils and fats, such as avocado oil, camellia oil, turtle oil, macadamia nut oil, corn oil, mink oil, olive oil, rapeseed oil, sesame oil, persic oil, wheat germ oil, camellia kissi seed oil, castor oil, linseed oil, safflower oil, cottonseed oil, perilla oil, soybean oil, peanut oil, tea seed oil, torreya nucifera seed oil, rice bran oil, China wood oil, Japanese tung oil, jojoba oil, germ oil, cocoa butter, coconut oil, hydrogenated coconut oil, palm oil, and Japan wax kernel oil; animal oils and fats, such as egg yolk oil, horse fat, tallow, mutton tallow, hydrogenated beef tallow, lard, beef bone fat, and neatsfoot oil; and the like.

### -Wax-

Examples of the wax include Japan wax, beeswax, candelilla wax, cotton wax, carnauba wax, bayberry wax, insect wax, spermaceti wax, montan wax, bran wax, lanolin, Ceiba pentandra wax, lanolin acetate, liquid lanolin, sugarcane wax, lanolin fatty acid isopropyl ester, hexyl laurate, reduced lanolin, jojoba wax, hard lanolin, shellac wax, and the like.

### -Hydrocarbon oil-

Examples of the hydrocarbon oil include straight-chain, branched-chain, or volatile hydrocarbon oil. Specific examples of the hydrocarbon oil include liquid paraffin, squalane, isoparaffin, α-olefin oligomer, polybutene, microcrystalline wax, hydrogenated polydecene, and the like.

### -Higher fatty acids-

Examples of the higher fatty acids include lauric acid, myristic acid, palmitic acid, stearic acid, behenic acid, oleic acid, 12-hydroxystearic acid, undecylenic acid, lanolin fatty acids, isostearic acid, linoleic acid, linolenic acid, eicosapentaenoic acid, and the like.

### -Higher alcohols-

Examples of the higher alcohols include alcohols with 6 or more carbon atoms. Specific examples of the higher alcohols include straight-chain or branched-chain higher alcohols, such as lauryl alcohol, cetyl alcohol, stearyl alcohol, behenyl alcohol, myristyl alcohol, oleyl alcohol, cetearyl alcohol, monostearyl glycerin ether (batyl alcohol), 2-decyltetradecynol, 2-octyldodecanol, lanolin alcohol, cholesterol, phytosterol, hexyldodecanol, isostearyl alcohol, octyldodecanol, and the like.

### -Ester oil-

Examples of the ester oil include glycerin triisooctanoate, glycerin triisopalmitate, octyl isopalmitate, isopropyl isostearate, isostearyl isostearate, isocetyl isostearate, hexyl isostearate, myristyl isostearate, isocetyl octanoate, cetyl isooctanoate, isostearyl octanoate, isodecyl isononanoate, octyldodecyl dimethyloctanoate, oleyl myristate, isostearyl erucate, isocetyl stearate, octyl stearate, isostearyl palmitate, isocetyl palmitate, octyl palmitate, isostearyl myristate, isocetyl myristate, octyldodecyl myristate, decyl myristate, isopropyl palmitate, isopropyl myristate, trimethylolpropane triisostearate, neopentyl glycol dicaprate, dioctyl sebacate, octyldodecyl 12-stearoyloxystearate, dioctyldodecyl stearoylglutamate, dioctyl adipate, diisostearyl malate, octyl methoxycinnamate, octyldodecyl lactate, isostearyl lactate, octyl paradimethylaminobenzoate, and the like.

### -Silicone oil-

Examples of the silicone oil include volatile cyclic silicone, volatile dimethyl polysiloxane, methyl phenyl silicone, and the like.

### -Oil-soluble drugs-

Examples of the oil-soluble drugs include oil-soluble vitamins, such as vitamin A, retinol, retinol acetate, retinol palmitate, benzyl nicotinate, dl-α-tocopherol nicotinate, vitamin D2, tocopherol, and the like.

### <<Polymers>>

Examples of the polymers include plant-based polymers, animal-based polymers, microbial polymers, starch-based polymers, cellulose-based polymers, alginic acid-based polymers, vinyl polymers, acrylic polymers, inorganic water-soluble polymers, and the like. The above polymers may function as thickeners.

Examples of the plant-based polymers include gum Arabic, tragacanth gum, galactan, guar gum, carob gum, karaya gum, gellan gum, carrageenan, pectin, agar, and the like.

Examples of the animal-based polymers include collagen, casein, albumin, gelatin, and the like.

Examples of the microbial polymers include xanthan gum, dextran, succinoglucan, pullulan, and the like.

Examples of the starch-based polymers include: plant starches, such as corn, wheat, potato, and rice; carboxymethyl starch; methylhydroxypropyl starch; and the like.

Examples of the cellulose-based polymers include methyl cellulose, nitrocellulose, ethyl cellulose methylhydroxypropyl cellulose, hydroxyethyl cellulose, sodium cellulose sulfate, hydroxypropyl cellulose, sodium carboxymethyl cellulose, crystalline cellulose, and the like.

Examples of the alginic acid-based polymers include sodium alginate, propylene glycol alginate, and the like.

Examples of the vinyl polymers include polyvinyl alcohol, polyvinyl acetate, polyvinyl methyl ether, polyvinyl pyrrolidone, vinyl pyrrolidone-vinyl acetate copolymers, carboxy vinyl polymers, and the like.

Examples of the acrylic polymers include sodium polyacrylate, polyethyl acrylate, alkanolamine polyacrylate, alkyl methacrylate-dimethylaminoethyl methacrylate copolymers, poly(2-acrylamido-2-methylpropane sulfonic acid), polymethacryloyloxy trimethylammonium, and the like.

Examples of the inorganic water-soluble polymers include polyethylene imine, cationic polymers, bentonite, magnesium aluminum silicate, laponite, hectorite, silicic anhydride, and the like.

### <<Surfactants>>

Examples of the surfactants include anionic surfactants, cationic surfactants, nonionic surfactants, amphoteric surfactants, and the like.

Examples of the anionic surfactants include soap base; fatty acid soap, such as sodium laurate and sodium palmitate; higher alkyl sulfate, such as sodium lauryl sulfate and potassium lauryl sulfate; alkyl ether sulfates, such as triethanol amine polyoxyethylene (may be abbreviated as "POE" hereinafter), triethanolamine lauryl sulfate, and sodium lauryl POE sulfate; N-acylsarcosine acid, such as sodium lauroylsarcosine; higher fatty acid amide sulfonic acid, such as sodium N-myristoryl-N-methyltaurate and sodium cocoyl methyl taurate; phosphate salts, such as POE stearyl ether phosphate; sulfosuccinates, such as monolauroylmonoethanolamide POE sulfosuccinate, and sodium lauryl polypropylene glycol sulfosuccinate; alkyl benzenesulfonates, such as sodium linear decyl benzene sulfonate and triethanolamine linear dodecyl benzene sulfonate; N-acylgultamate, such as disodium N-stearoylglutamate and monosodium N-stearoylglutamate; higher fatty acid ester sulfate salts, such as sodium hydrogenated cocoate sulfate; sulfonated oil, such as sulfonated castor oil; POE alkyl ether carboxylic acid; POE alkyl allyl ether carboxylate; higher fatty acid ester sulfonate; secondary alcohol sulfate; higher fatty acid alkylolamide sulfate; sodium lauryl monoethanolamide succinate; and the like.

Examples of the cationic surfactants include: alkyl trimethyl ammonium salts, such as stearyl trimethyl ammonium chloride and lauryl trimethyl ammonium chloride; dialkyldimethyl ammonium salts, such as distearyldimethyl ammonium chloride; alkyl pyridinium salts, such as cetylpyridinium chloride; alkyl quaternary ammonium salts; alkyldimethylbenzyl ammonium salts; alkylisoquinolinium salts; dialkylmorpholinium salts; POE alkylamine; alkylamine salts; polyamine fatty acid derivatives; amyl alcohol fatty acid derivatives; benzalkonium chloride; and the like.

Examples of the nonionic surfactants include lipophilic nonionic surfactants, hydrophilic nonionic surfactants, and the like. Examples of the lipophilic nonionic surfactants include: sorbitan fatty acid esters, such as sorbitan monooleate, sorbitan monoisostearate, sorbitan monolaurate, sorbitan monopalmitate, and sorbitan trioleate; glycerin polyglycerin fatty acids, such as cottonseed oil monoglyceride, glycerol monostearate, glyceryl sesquioleate, and glyceryl monostearate/malate; propylene glycol fatty acid esters, such as propylene glycol monostearate; glycerin alkyl ether; POE-methyl polysiloxane copolymers; and the like. Examples of the hydrophilic nonionic surfactants include: POE sorbitan fatty acid esters, such as POE sorbitan monooleate and POE sorbitan monostearate; POE sorbit fatty acid esters, such as POE sorbit monolaurate, POE sorbit monolaurate, and POE sorbit monostearate; POE glycerin fatty acid esters, such as POE glycerin monooleate and POE glycerin distearate; POE fatty acid esters, such as POE monooleate, POE distearate, and POE monodioleate; POE alkyl ethers, such as POE lauryl ether, POE oleyl ether, and POE cholestanol ester; POE alkylphenyl ethers, such as POE octylphenyl ether and POE nonylphenyl ether; POE/polyoxypropylene (may be abbreviated as "POP") alkyl ethers, such as POE/POP monobutyl ether, POE/POP cetyl ether, and POE/POP glycerin ether; POE beeswax/lanolin derivatives, such as POE sorbit beeswax; alkanol amide, such as coconut fatty acid diethanolamide and fatty acid isopropanolamide; POE propylene glycol fatty acid esters; POE fatty acid amide; POE alkylamine; sucrose fatty acid esters; alkylethoxydimethylamine oxide; and the like.

Examples of the amphoteric surfactants include: imidazoline-based amphoteric surfactants, such as 2-cocoyl-2-imidazoliniumhydroxide-1-carboxyethyloxy disodium salt; betaine-based surfactants, such as amidobetaine and sulfobetaine; and the like.

### <<Powders>>

The powders may be inorganic powders or organic powders.

Examples of the inorganic powders include mica, talc, kaolin, sericite (sericite), muscovite, phlogopite, synthetic mica, lepidolite, biotite, lithia mica, synthetic mica, silicic anhydride (silica), aluminum silicate, barium silicate, calcium silicate, magnesium silicate, strontium silicate, aluminum oxide, barium sulfate, zeolite, titanium oxide, zinc oxide, boron nitride, and the like.

Examples of the organic powders include polyamide resin powders (nylon powders), polyethylene powders, poly(methyl methacrylate) powders, polystyrene powders, calcium carbonate powders, magnesium carbonate powders, styrene-acrylic acid copolymer powders, cellulose powders, and the like.

### <<Colorants>>

Examples of the colorants include pigments and dyes.

### -Pigments-

Examples of the pigments include inorganic pigments, organic pigments, pearl pigments, metal powder pigments, and the like.

Examples of the inorganic pigments include: inorganic white pigments, such as titanium dioxide and iron oxide (red iron oxide); inorganic yellow pigments, such as yellow iron oxide and ochre; black pigments, such as black iron oxide and carbon black; inorganic green pigments, such as chromium oxide, chromium hydroxide, and cobalt titanate; inorganic blue pigments, such as ultramarine and Prussian blue; and the like.

Examples of the organic pigments include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 202, Yellow No. 203, Yellow No. 205, Yellow No. 401, Blue No. 1, Blue No. 404, Green No. 3, zirconium lakes of the foregoing, barium lakes of the foregoing, and aluminum lakes of the foregoing.

Examples of the pearl pigments include titanium oxide-coated mica, colored titanium oxide-coated mica, bismuth oxychloride, fish scale flakes, and the like.

Examples of the metal powder pigment include aluminum powders, copper powders, and the like.

### -Dyes-

Examples of the dyes include natural dyes, such as chlorophyll, β-carotene, and the like.

### <<Solvents>>

Examples of the solvents include water, lower alcohols, and the like. Examples of the lower alcohols include alcohols with 5 or less carbon atoms. Specific examples of the lower alcohols include methanol, ethanol, propyl alcohol, isopropyl alcohol, 2-amino-2-methyl-1-propanol, 2-amino-2-methyl-1,3-propanediol, and the like.

### <<Silicones>>

The silicones are not particularly limited, and may be appropriately selected according to the intended purpose. Examples of the silicones include chain silicones, cyclic silicone, modified silicones, and the like.

Examples of the chain silicones include dimethyl polysiloxane (dimethicone), methylphenyl polysiloxane (phenyl methicone), methyl hydrogen polysiloxane, and the like.

Examples of the cyclic silicone include octamethylcyclotetrasiloxane, decamethylcyclopentasiloxane, dodecamethylcyclohexasiloxane, and the like.

Examples of the modified silicones include amino-modified silicone oil, polyether-modified silicone oil, carboxy-modified silicone oil, alkyl-modified silicone oil, ammonium salt-modified silicone oil, fluorine-modified silicone oil, and the like.

The above silicones may be used alone or in combination of two or more.

### <<Drugs or medical ingredients>>

The drugs are not particularly limited as long as the drugs are drugs or medical ingredients other than the puffiness-reducing agent. Examples of the drugs or medical ingredients include vitamins, ultraviolet-ray absorbers, chelating agents, preservatives, plant extracts, moisturizing agents, anti-inflammatory agents, whitening agents, cooling agents, amino acids, antioxidants, bactericides, various skin nutrients, and the like.

### -Vitamins-

Examples of the vitamins include water-soluble vitamins, such as vitamin B6 hydrochloride, pantothenyl ethyl ether, pyridoxine hydrochloride, nicotinic acid amide, pantothenic acid, biotin, and the like.

### -Ultraviolet-ray absorbers-

Examples of the ultraviolet-ray absorbers include: benzoic acid-based ultraviolet-ray absorbers, such as p-aminobenzoic acid; anthranilic acid-based ultraviolet-ray absorbers, such as methyl anthranilate; salicylic acid-based ultraviolet-ray absorbers, such as octyl salicylate; cinnamic acid-based ultraviolet-ray absorbers, such as isopropyl p-methoxycinnamate and octyl p-methoxycinnamate; ultraviolet-ray absorbers, such as urocanic acid and ethyl urocanate; benzophenone-based ultraviolet-ray absorbers, such as 2-hydroxy-4-methoxybenzophenone and dihydroxybenzophenone; benzotriazole-based ultraviolet-ray absorbers; 2-phenylbenzimidazole-5-sulfonic acid; and the like.

### -Chelating agents-

Examples of the chelating agents include citramalic acid, argaric acid, glyceric acid, shikimic acid, hinokitiol, gallic acid, tannic acid, caffeic acid, ethylenediamine-N,N,N',N'-tetraacetic acid disodium salt dihydrate (EDTA-2Na-2H₂O₂), ethylene glycol diaminetetraacetic acid, diethylenetriaminepentaacetic acid, phytic acid, polyphosphoric acid, metaphosphoric acid, analogs of the foregoing, alkali metal salts of the foregoing, carboxylic acid esters of the foregoing, and the like.

### -Preservatives-

Examples of the preservatives include benzoic acid, salicylic acid, p-oxybenzoic acid ester (e.g., methylparaben, ethylparaben, butylparaben, etc.), sorbic acid, p-chlorometacresol, hexachlorophene, benzalkonium chloride, chlorohexidine chloride, trichlorocarbanilide, photosensitizers, phenoxyethanol, chlorphenesin, and the like.

### -Plant extracts-

Examples of the plant extracts include Houttuynia cordata extracts, Phellodendron amurense extracts, licorice extracts, Paeonia Lactiflora extracts, Paeonia Suffruticosa extracts, Luffa Cylindrica extracts, Saxifraga Sarmentosa extracts, eucalyptus extracts, clove extracts, horse chestnut extracts, Centaurea Cyanus Flowe extracts, seaweed extracts, thyme extracts, and the like. The above extracts may include caffeine, tannin, and the like.

### -Moisturizing agents-

Examples of the moisturizing agents include polyethylene glycol (may be abbreviated as "PEG" hereinafter), sodium chondroitin sulfate, sodium hyaluronate, sodium lactate, glucosamine, cyclodextrin, and the like. In addition, the glycols, the glycerins, the sugar alcohols, and the saccharides can be also used as the moisturizing agents.

### -Anti-inflammatory agents-

Examples of the anti-inflammatory agents include azulene, glycyrrhizin, lysozyme chloride, pyridoxine hydrochloride, sulfur, and the like.

### -Whitening agents-

Examples of the whitening agents include arbutin, 4-methoxysalicylic acid, tranexamic acid, vitamin C, ethyl vitamin C, magnesium ascorbyl phosphate, ascorbyl glucoside, kojic acid, and the like.

### -Cooling agents-

Examples of the cooling agents include L-menthol, camphor, and the like.

### -Antioxidants-

Examples of the antioxidants include ascorbic acid, α-tocopherol, carotenoid, and the like.

### <<pH Adjuster>>

Examples of the pH adjuster include potassium hydroxide, sodium hydroxide, triethanolamine, sodium carbonate, lactic acid, citric acid, sodium citrate, glycolic acid, succinic acid, tartaric acid, malic acid, sodium hydrogen carbonate, ammonium hydrogen carbonate, and the like.

### <<Sequestering agents>>

Examples of the sequestering agents include disodium ethylenediaminetetraacetate, trisodium ethylenediaminetetraacetate, sodium citrate, sodium polyphosphate, sodium metaphosphate, gluconic acid, and the like.

The amount of the other ingredients in the cosmetic according to one embodiment is not particularly limited, and may be appropriately selected according to the intended purpose, as long as the effect of the cosmetic according to one embodiment is not impaired.

### [Use]

Use of the cosmetic is not particularly limited, and may be appropriately selected according to the intended purpose. Examples of use include lotions, emulsified lotions, creams, essences, jelly, gels, ointments, packs, masks, foundations, and the like.

### Examples

Hereinafter, the embodiment will be more concretely described through Example, Comparative Example, and Test Example, but the embodiment is not limited to Example, Comparative Example, and Test Example. In Example and Comparative Example presented in Table 1 below, the blending amount is represented by "percent by mass" relative to the total mass of the lotion.

### (Example 1)

A lotion having the composition and the blending amounts presented in Table 1 was prepared according to a common procedure.

### (Comparative Example 1)

A lotion having the composition and the blending amounts presented in Table 1 was prepared according to a common procedure.

**[Table 1]**

| Composition (mass%) | | Ex. 1 | Comp. Ex. 1 |
|---|---|---|---|
| Puffiness-reducing agent | 1-(2-hydroxyethyl)-2-imidazolidinone | 1.50 | - |
| Moisturizing agent | dipropylene glycol | 5.00 | 5.00 |
| | 1,3-butylene glycol | 5.00 | 5.00 |
| Thickener | carboxy vinyl polymer | 0.13 | 0.13 |
| pH Adjuster | potassium hydroxide | 0.04 | 0.04 |
| Chelating agent | EDTA-2Na-2H₂O₂ | 0.02 | 0.02 |
| Preservative | chlorphenesin | 0.20 | 0.20 |
| | phenoxyethanol | 0.50 | 0.50 |
| Water | ion-exchanged water | remaining | remaining |
| Total (mass%) | | 100.00 | 100.00 |

### (Test Example 1)

Thirty-nine healthy Japanese female panelists in their 20s to 60s were asked to apply 0.3 mL of the lotion of Example 1 to a half of the face twice a day, in the morning and at night. The half of the face, whether left or right, was randomly assigned, 19 panelists applied the lotion of Example 1 onto the right side with respect to the median line of the face, and 20 panelists applied the lotion of Example 1 onto the left side with respect to the median line of the face. The half of the face to which the lotion of Example 1 was applied was determined as a "Q half face". Next, the same panelists were asked to apply 0.3 mL of the lotion of Comparative Example 1 to the other half of the face opposite to the half on which the lotion of Example 1 had been applied, twice a day, in the morning and at night. The other half of the face of the same panelist opposite to the half to which the lotion of Example 1 was applied means as follows. For example, the panelist who had applied the lotion of Example 1 to the left side with respect to the median line of the face applied the lotion of Comparative Example 1 to the right side with respect to the median line of the face. The half of the face to which the lotion of Comparative Example 1 was applied was determined as a "P half face". The application of the lotions of Example 1 and Comparative Example 1 was carried out for 6 weeks. The compositions of the lotions of Example 1 and Comparative Example 1 were not disclosed to the panelists. Next, the following visual sensory determination and quantification evaluation were performed.

### <Visual sensory determination>

Left side and right side facial photographs were taken for the half of the face to which the lotion of Example 1 was applied (Q half face) and the half of the face to which the lotion of Comparative Example 1 was applied (P half face) by a digital camera from the positions at an angle of 45 degrees with respect to the front, when the median line of the face was determined as the front. The left and right face photographs were used as a pair, and four professional evaluators performed visual sensory determination as to "which side of the face was puffy, left or right". Fig. 3A is a schematic explanatory diagram of a pair of the left and right face photographs. The visual sensory determination was performed without disclosing the information regarding to which half faces the lotions of Example 1 Comparative Example 1 had been applied, respectively, to the professional evaluators.

The determination was performed before the initial application of the lotions o Example 1 and Comparative Example 1 (referred to as "Week 0" hereinafter), and 3 weeks and 6 weeks after the start of the application of the lotions of Example 1 and Comparative Example 1. For the determination of each week, each of four professional evaluators performed the determination 5 times. The determination was performed based on the following evaluation criteria. Each professional evaluator gave evaluation points to the Q half face and the P half face, respectively, and for the determination of each week, the average value a of the evaluation points from the determination performed 5 times per professional evaluator was calculated for each panelist. Next, the average value b of the average values a of 39 panelists was calculated for each of at Week 0, after 3 weeks, and after 6 weeks. Next, the average value c of the average values b of the four professional evaluators was evaluated. In addition, the standard error of the average value b was calculated.

### -Determination criteria-

Evaluation point -1: The Q half face looked puffy.
Evaluation point 1: The P half face looked puffy.

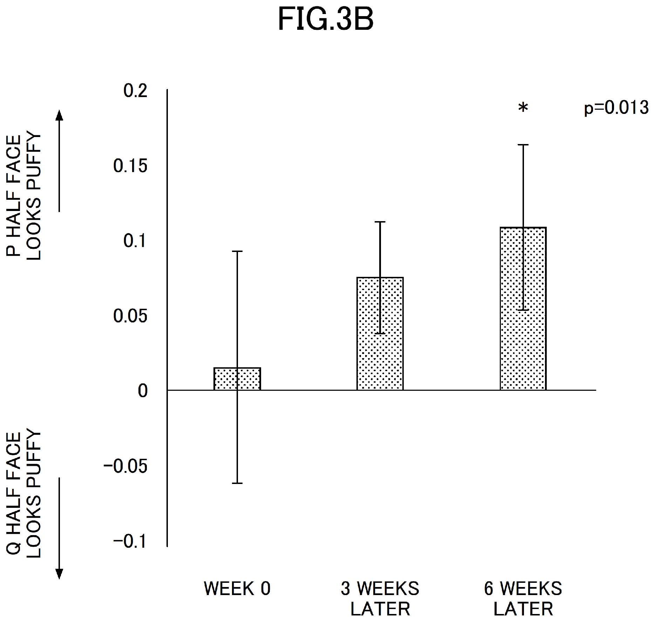

The results are presented in Fig. 3B. In Fig. 3B, "*" indicates that the p value was 0.013 according to the pared-T test (two-tailed T test) performed on the result before application (Week 0) and the result after 6 weeks, and there was a significant difference. There was no difference between the P half face and the Q half face before the application (Week 0). Conversely, the result that the P half face looked significantly puffy, in other words, that the Q half face looked less puffy, was obtained after 6 weeks from the start of the application.

### <Quantitative evaluation>

The data (three-dimensional face shape image) representing the three-dimensional shape of the face of the panelist was captured by a 3D imaging analysis device (VECTRA Handy H2 (VEC-H2, manufactured by Canfield Scientific, Inc.) before the initial application of the lotions of Example 1 and Comparative Example 1, and after 3 weeks and 6 weeks from the start of the application of the lotions of Example 1 and Comparative Example 1. At the time of capturing the data, the three-dimensional face shape image with the face of the panelist in the horizontal position (specifically, the median line of the face was fixed to be orthogonal to the direction of gravity) and the three-dimensional face shape image with the face of the panelist in the vertical position (specifically, the median line of the face was fixed to be parallel to the direction of gravity) were both obtained. Next, an amount of gravitational swelling (VCswelling) and an amount of gravitational shrinking (VCshrinking) for each of the half of the face to which the lotion of Example 1 was applied (Q half face) and the half of the face to which the lotion of Comparative Example 1 was applied (P half face) were calculated by a computer based on a three-dimensional shape difference between the three-dimensional face shape image with the face of the panelist in the horizontal position and the three-dimensional face shape image with the face of the panelist in the vertical position. Next, the following amount of puffiness (Sc) of the face was calculated based on the amount of gravitational swelling (VCswelling) and the amount of gravitational shrinking (VCshrinking).

### -Calculation of amount of puffiness (Sc) of face-

The amount of puffiness (Sc) of the face was calculated according to the following equation (1). Three three-dimensional face shape images were acquired per panelist each of at Week 0, after 3 weeks, and after 6 weeks, and the average value was determined as the amount of puffiness (Sc) of the face of the panelist.
[Math. 2] ${S}_{c} = \frac{1}{\sqrt{2}} \left({VC}_{swelling}-{VC}_{shirinking}\right)$ Equation (1) (In equation (1), "Sc" is an amount of puffiness of face, "VCswelling" is an amount of gravitational swelling, and "VCshrinking" is an amount of gravitational shrinking.)

Next, the reduction in the amount of puffiness (Sc) of the face was calculated according to the following equations (3) and (4). 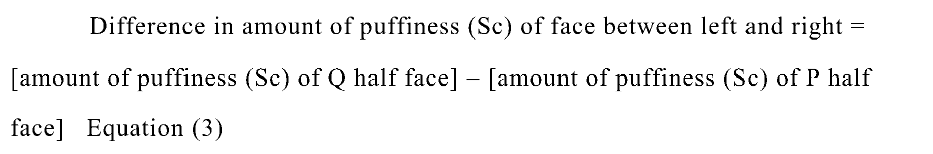Difference in amount of puffiness (Sc) of face between left and right = [amount of puffiness (Sc) of Q half face] - [amount of puffiness (Sc) of P half face] Equation (3) 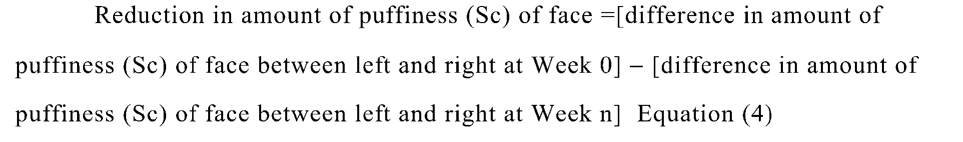 Reduction in amount of puffiness (Sc) of face =[difference in amount of puffiness (Sc) of face between left and right at Week 0] - [difference in amount of puffiness (Sc) of face between left and right at Week n] Equation (4) (In the equation (4), n = 3 or 6)

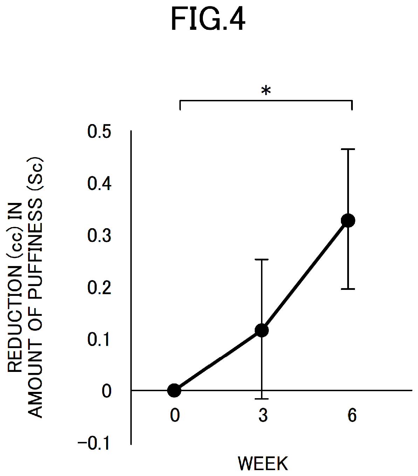

The graph representing the change in reduction of the amount of puffiness (Sc) of the face between Week 0, which was the start of the test, and after 6 weeks is depicted in Fig. 4. In Fig. 4, "*" indicates p < 0.05. The result demonstrates that the p value between Week 0 and after 6 weeks was less than 0.05, and the amount of the puffiness (Sc) of the Q half face to which the lotion of Example 1 was applied was significantly reduced compared to that of the P half face to which the lotion of Comparative Example 1 was applied.

It was found from the results of Test Examples 1 and 2 that the puffiness-reducing agent including, as an active ingredient, 1-(2-hydroxyethyl)-2-imidazolidinone or a derivative of 1-(2-hydroxyethyl)-2-imidazolidinone had an excellent puffiness reduction effect.

Embodiments of the present invention include, for example, the following.
<1> A puffiness-reducing agent includes an active ingredient that is 1-(2-hydroxyethyl)-2-imidazolidinone or a derivative of 1-(2-hydroxyethyl)-2-imidazolidinone.
<2> In the puffiness-reducing agent according to <1>, the 1-(2-hydroxyethyl)-2-imidazolidinone or the derivative of 1-(2-hydroxyethyl)-2-imidazolidinone includes, as an active ingredient, a cyclic carboxamide derivative represented by a general formula (1) below or a salt of the cyclic carboxamide derivative, 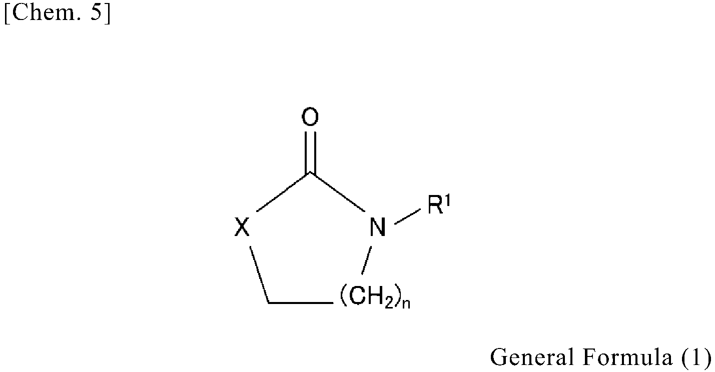 (in the general formula (1), n is an integer of 1 to 3, R¹ is a hydrogen atom or a hydrocarbon group that includes 1 to 6 carbon atoms and may be substituted with a hydroxyl group, and X is a group represented by -CH₂- or -N(R²)-, where R² is a hydrogen atom or a hydrocarbon group that includes 1 to 6 carbon atoms and may be substituted with a hydroxyl group).
<3> The puffiness-reducing agent according to <1> or <2> is a topical dermatological agent.
<4> A cosmetic includes the puffiness-reducing agent according to any one of <1> to <3>.
<5> 1-(2-Hydroxyethyl)-2-imidazolidinone or a derivative of 1-(2-hydroxyethyl)-2-imidazolidinone for use in reduction of puffiness.
<6> Use of 1-(2-hydroxyethyl)-2-imidazolidinone or a derivative of 1-(2-hydroxyethyl)-2-imidazolidinone in production of a puffiness-reducing agent.

As described above, the present invention has been described based on the specific embodiments and examples, but these embodiments and examples are merely presented as examples, and the present invention is not limited to the above embodiments and examples. The above embodiments can be implemented in various other forms, and various combinations, omissions, substitutions, additions, changes, and the like can be made without departing from the gist of the invention. These embodiments and modifications are included in the scope and gist of the invention, and are included in the invention described in the claims and the equivalent scope of the claims.

The present international application claims priority based on Japanese Patent Application No. 2023-220018, filed on December 26, 2023, and the entire contents of Japanese Patent Application No. 2023-220018 are incorporated herein by reference.

## Claims

1. A puffiness-reducing agent comprising:
an active ingredient that is 1-(2-hydroxyethyl)-2-imidazolidinone or a derivative of 1-(2-hydroxyethyl)-2-imidazolidinone.

2. The puffiness-reducing agent according to claim 1,
wherein the 1-(2-hydroxyethyl)-2-imidazolidinone or the derivative of 1-(2-hydroxyethyl)-2-imidazolidinone includes, as an active ingredient, a cyclic carboxamide derivative represented by a general formula (1) below or a salt of the cyclic carboxamide derivative, 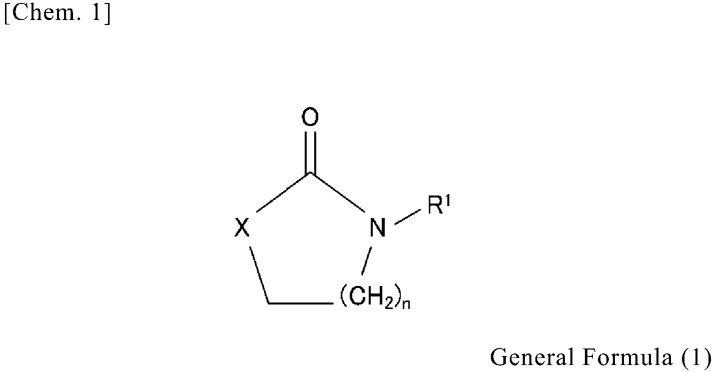 (in the general formula (1), n is an integer of 1 to 3, R¹ is a hydrogen atom or a hydrocarbon group that includes 1 to 6 carbon atoms and may be substituted with a hydroxyl group, and X is a group represented by -CH₂- or -N(R²)-, where R² is a hydrogen atom or a hydrocarbon group that includes 1 to 6 carbon atoms and may be substituted with a hydroxyl group).

3. The puffiness-reducing agent according to claim 1,
wherein the puffiness-reducing agent is a topical dermatological agent.

4. A cosmetic comprising:
the puffiness-reducing agent of claim 1.
